# FASCICULE DE BREVET EUROPEEN

(11) **EP 1 397 504 B1**
(45) Date de publication et mention de la délivrance du brevet: **12.04.2006**
(21) Numéro de dépôt: 02747530.0
(22) Date de dépôt: 13.06.2002
(51) Int. Cl.: C12Q 1/04, C12Q 1/10, C12Q 1/34

(54) **MILIEU DE CULTURE POUR LA DETECTION ET/OU LA DISCRIMINATION DES ENTEROCOQUES**
NÄHRBODEN ZUM NACHWEIS UND/ODER ZUR UNTERSCHEIDUNG VON ENTEROKOKKEN
CULTURE MEDIUM FOR DETECTING AND/OR DISCRIMINATING ENTEROCOCCUS AND METHOD THEREFOR

(30) Priorité: 13.06.2001 FR 0107730
(43) Date de publication de la demande: 17.03.2004
(73) Titulaire: Rambach, Alain, F-75006 Paris (FR)
(72) Inventeur: Rambach, Alain, F-75006 Paris (FR)
(74) Mandataire: Warcoin, Jacques
(86) Numéro de dépôt international: PCT/FR2002/002025
(87) Numéro de publication internationale: WO 2002/101082

(56) Documents cités:
- WO-A-01/06000
- DE-A- 3 730 534
- US-A- 5 443 963
- DATABASE BIOSIS [en ligne] BIOSCIENCES INFORMATION SERVICE, PHILADELPHIA, PA, US; 1983 BRAYTON P R ET AL: "NEW SELECTIVE PLATING MEDIUM FOR ISOLATION OF VIBRIO-VULNIFICUS BIOGROUP 1" Database accession no. PREV198376057962 XP002200146 & JOURNAL OF CLINICAL MICROBIOLOGY, vol. 17, no. 6, 1983, pages 1039-1044, ISSN: 0095-1137
- DATABASE BIOSIS [en ligne] BIOSCIENCES INFORMATION SERVICE, PHILADELPHIA, PA, US; 1978 MOATS W A ET AL: "EFFECT OF PH ON THE ANTI MICROBIAL ACTIVITY OF SOME TRI PHENYL METHANE DYES" Database accession no. PREV197866050556 XP002200147 & CANADIAN JOURNAL OF MICROBIOLOGY, vol. 24, no. 6, 1978, pages 658-661, EN ISSN: 0008-4166

## Description

La présente invention concerne un milieu de culture chromogène destiné à mettre en évidence les entérocoques.

Dans le domaine clinique, la détection des entérocoques s'avère extrêmement importante, en raison de l'apparition de souches résistantes aux antibiotiques, notamment à la vancomycine, l'antibiotique le plus utilisé pour traiter les infections. Ces infections nosocomiales peuvent ainsi mettre en danger la vic des patients atteints.

Les streptocoques isolés de l'intestin ou streptocoques du groupe D étaient classiquement distingués en deux groupes selon leurs caractéristiques physiologiques :
- les "entérocoques" (*Streptococcus faecium* et *Streptococcus faecalis*) capables de croître dans des conditions hostiles ;
- les "streptocoques du groupe D non-entérocoques" *(Streptococcus bovins et Streptococcus equinus)* incapables de se multiplier dans des conditions hostiles.

En 1982 et en 1983, les études génomiques (hybridations AND - ARNr. établissement de dictionnaires des oligonucléotides de l'ARNr 16S) ont montré que les "entérocoques" et les "streptocoques du groupe D non-entérocoques" appartenaient à deux genres différents.

En 1984, Schleifer et Kilpper-Bälz en étudiant les résultats des hybridations ADN - ARNr 23S et des hybridations ADN - ADN confirment les résultats antérieurs et ces deux auteurs proposent le transfert des "streptocoques du groupe des entérocoques" dans le genre *Enterococcus,* distinct du genre *Streptococcus* (*Int*. *J. Sysl. Bacteriol.,* 1984, **34**, 31-34).

Une très bonne étude sur les entérocoques peut être trouvée sur le site www.bacterio.cict.fr/bacdico/ee/enterococcus.html, ou sur le site www.life.umd.edu/classroom/bsci424/PathogenDescriptions/Enterococcus.htm, ou sur le site www.enterococcus.ouhsc.edu/lab_methods.asp.

Il est donc important de disposer d'un test fiable et rapide permettant de détecter les contaminations par ces bactéries, lequel test devant être à la fois sensible et spécifique.

Les entérocoques croissent de façon générale dans des gammes de température de 10° à 45°C, la croissance optimale étant autour de 35°C, sur milieu non sélectif (agar à base de sang ou agar chocolat). Toutefois, un tel milieu permet également la croissance d'autres bactéries et ne permet pas de distinguer effectivement les entérocoques.

Il existe aujourd'hui certains milieux de culture pour la détection des entérocoques, notamment le milieu MacConkey Agar, sans Cristal Violet, vendu par Difco. Le Cristal Violet est un composé inhibant la croissance des bactéries Gram positives, dont on considère qu'il inhibe la croissance des entérocoques et des staphylocoques (rappelons que les entérocoques sont des coques à Gram positif, se présentant de manière isolée ou en paires ou en courtes chaînes). Le « Difco Manual », 11^{ème} édition, page 288, indique « MacConkey Agar w/o CV (Crystal Violet) is a differential medium that is less selective than MacConkey Agar. The lack of crystal violet permits the growth of *Staphylococcus* and *Enterococcus.* »

Ce milieu contient également d'autres inhibiteurs de croissance, tels des sels biliaires. Ces autres inhibiteurs sont efficaces pour inhiber la croissance de bactéries Gram positives, mais pas des entérocoques.

En effet, les bactéries entérocoques peuvent également croître dans des milieux contenant des sels biliaires, les colonies ne se dissolvant pas après exposition à la bile. On peut aussi faire croître les bactéries entérocoques dans un milieu contenant une concentration de 6,5% en NaCl, les streptocoques ne possédant pas cette propriété.

De ces constatations que les milieux pour entérocoques ne contiennent pas de Cristal Violet, mais contiennent plutôt d'autres inhibiteurs, on peut conclure que, dans de nombreuses conditions, le Cristal Violet est un inhibiteur de croissance des entérocoques. Cette hypothèse est renforcée par le fait que parmi les différents milieux MacConkey, celui utilisé pour détecter les entérocoques ne contient pas de Cristal Violet, mais contient des sels biliaires. Toutefois, ce milieu permet aussi la croissance des staphylocoques.

Il convient de noter que la méthode la plus couramment utilisée pour la détection des entérocoques consiste en une technique de filtration sur un milieu sélectif (tel que le milieu m-*Enterococcus* agar = milieu de Slanetz et Bartley ou le milieu acide oxolinique-esculine-azide = milieu OAA) suivie d'une confirmation par culture sur une gélose bile-esculine incubée 48 heures à 44 °C. L'effet sélectif peut être renforcé en incubant le milieu de Slanetz et Bartley à 41 °C. Le plus souvent, l'identification complète n'est pas réalisée et seule la recherche de la catalase est effectuée afin d'éliminer certaines souches de staphylocoques capables de se développer sur les milieux utilisés.

Il convient donc de disposer d'un milieu permettant la détection des entérocoques qui permette également de les différentier des staphylocoques, qui poussent en général sur les milieux de culture des entérocoques.

Dans le cadre de la présente invention, il a été montré qu'il est possible d'ajuster la concentration de Cristal Violet de telle manière que celui-ci garde son rôle d'inhibition de croissance pour la plupart des bactéries Gram positives, et notamment des staphylocoques, tout en permettant la croissance des entérocoques.

Ainsi, les milieux de l'art antérieur (notamment le milieu MacConkey Agar vendu par les laboratoires Difco) utilisent généralement une concentration de Cristal Violet égale à 1 mg/l. Il a été montré que l'ajout de Cristal Violet à une concentration de 0,1 jusqu'à environ 1,5 mg/l permet de maintenir la croissance des entérocoques, notamment lorsque le milieu ne contient pas d'autres inhibiteurs de croissance pour bactéries Gram positives.

Il a également été montré que l'ajout de Cristal Violet permet d'éviter d'utiliser de l'azide de sodium très souvent présent dans les milieux de détection des entérocoques (pour ses propriétés inhibitrices de croissance de certaines bactéries, mais qui est toxique pour l'homme). De façon préférée, un milieu selon l'invention ne contiendra donc pas d'azide de sodium.

Ce résultat, et notamment le fait que la concentration en Cristal Violet puisse être supérieure à 1 mg/l dans certaines conditions tout en permettant la croissance des entérocoques, ne pouvait être prévu à la lumière des connaissances décrites dans l'art antérieur, notamment que l'on puisse également supprimer l'azide de sodium.

Ainsi, la présente invention a pour objet un milieu de culture pour la détection et/ou la discrimination des entérocoques, caractérisé en ce qu'il comporte, dans un milieu de culture pour entérocoques, du Cristal Violet à une concentration permettant la croissance des entérocoques et l'inhibition de croissance de la plupart des bactéries Gram positives.

Ladite concentration est supérieure à 0,1 mg/l, de façon plus préférée, supérieure à 0,25 mg/l, ou supérieure à 0,5 mg/l. Ainsi, l'on observe une inhibition de croissance d'une quantité importante de bactéries Gram positives après ajout de Cristal Violet à une concentration aussi basse que 0,1 mg/l.

Ladite concentration est inférieure à 1 mg/l. Lorsque la concentration en Cristal Violet est à environ 1 mg/l, il convient alors de réduire, voire de supprimer les autres inhibiteurs de bactéries Gram positives, notamment les sels biliaires. Il est à la portée de l'homme du métier que d'ajuster les concentrations en inhibiteurs en fonction de la concentration en Cristal Violet ajouté dans le milieu selon l'invention.

Dans un autre mode de réalisation de l'invention, la concentration en Cristal Violet est inférieure à 0,8 mg/l, de façon plus préférée, inférieure à 0,7 mg/l. A de telles concentrations, on peut rajouter éventuellement d'autres inhibiteurs de bactéries Gram positives.

L'homme du métier sait ce que signifie le terme « milieu de culture pour entérocoques », c'est-à-dire un milieu de culture contenant les nutriments nécessaires pour permettre la croissance de ces bactéries. On cite notamment de la peptone de caséine, de soja, de viande, de l'extrait de levure ou de boeuf, du dextrose...

De façon préféré, le milieu selon l'invention comprend en outre au moins un agent chromogène, substrat d'une enzyme de fermentation des sucres, ladite enzyme étant, de préférence, une glucosidase, notamment la β-glucosidase ou une galactosidase, notamment la β-galactosidase.

Le fait que l'on puisse ajouter un agent chromogène est tout à fait inattendu, dans la mesure où le Cristal Violet colore déjà les milieux selon l'invention, ce qui dissuade donc de rajouter un élément apportant une couleur, tel un agent chromogène.

De préférence, ledit agent chromogène libère, par hydrolyse, un chromophore précipitable choisi parmi les dérivés indoxyle, halogéno-indoxyle (bromo-indoxyle, chloro-indoxyle, fluoro-indoxyle, iodo-indoxyle, dichloro-indoxyle, chloro-bromo- indoxyle, tri-chloro-indoxyle), méthyl-indoxyle, ou hydroxy-quinoline, en particulier les dérivés suivants : 6-chloro-indoxyle, 5-bromo-indoxyle, 3-bromo-indoxyle, 6- fluoro-indoxyle, 5-iodo-indoxyle, 4,6-dichloro-indoxyle, 6,7-dichloro-indoxyle, 5-bromo-4-chloro-indoxyle, 5-bromo-6-chloro-indoxyle, 4,6,7-trichloro-indoxyle, N-méthyl-indoxyle ou 8-hydroxy-quinoline.

De préférence, ledit agent chromogène substrat de la β-glucosidase est un indoxyl-glucoside, notamment le 5-bromo-4-chloro-3-indoxyl-β-glucoside et/ou ledit agent chromogène substrat de la β-galactosidase est un indoxyl-galactoside, en particulier le 5-bromo-6-chloro-3-indoxyl-β-galactoside.

Afin de mieux permettre la détection des entérocoques, on peut également rajouter, dans le milieu de culture selon l'invention, des inhibiteurs de croissance pour les bactéries Gram négatives, tels l'acide nalidixique ou la colistine.

Afin de détecter les entérocoques atypiques résistants à certains antibiotiques, et responsables en grande partie des infections nosocomiales, on peut aussi rajouter lesdits antibiotiques dans les milieux selon l'invention. On rajoutera ainsi de la vancomycine à une concentration d'environ 6 mg/l. Notons que l'on peut détecter la proportion de bactéries résistantes dans un échantillon en effectuant un étalement sur une boîte sans antibiotiques et une boîte les comprenant et en comparant le nombre de colonies identifiées comme entérocoques sur chaque boîte.

L'invention se rapporte également à l'utilisation d'un milieu de culture selon l'invention pour la détection et/ou la discrimination des entérocoques, ainsi qu'à un procédé de détection et/ou discrimination des entérocoques dans un échantillon, caractérisé en ce qu'il comprend les étapes consistant à :
a. inoculer un milieu de culture selon l'invention avec ledit échantillon ou un inoculum dérivé de l'échantillon,
b. détecter la présence d'entérocoques sur ledit milieu de culture.

On détecte la présence des entérocoques par la croissance des colonies sur le milieu, et l'on est aidé par leur coloration après libération du chromophore à partir du chromogène substrat de l'enzyme.

On choisit de préférence un chromophore ayant une longueur d'onde différente de la longueur d'onde du Cristal Violet, afin de l'identifier plus facilement. Toutefois, le milieu selon l'invention permet également l'utilisation et la détection de chromophores ayant une longueur d'onde proche de la longueur d'onde du Cristal Violet.

D'une façon générale, l'invention se rapporte aussi à un milieu de culture pour la détection de bactéries Gram positives ou Gram négatives, comprenant, outre des facteurs de croissance pour lesdites bactéries Gram positives ou Gram négatives, un agent chromogène et du Cristal Violet, le Cristal Violet étant présent à une concentration permettant la croissance desdites bactéries que l'on cherche à détecter et l'inhibition différentielle de croissance des bactéries Gram positives, ladite concentration étant comprise de préférence entre 0,1 et 1,0 mg/l.

L'invention a donc démontré que l'on peut ajouter un colorant dans un milieu chromogène, tout en gardant les propriétés du chromogène.

Le chromogène est choisi de telle sorte que le chromophore libéré possède une longueur d'onde différente de celle du Cristal Violet ou du colorant utilisé dans le milieu chromogène.

### EXEMPLES

### Exemple 1

Un milieu préféré pour la mise en oeuvre de l'invention comprend (pour un litre) :
- Agar 15 g
- Extrait de levure et peptones 9 g
- NaCl 5 g
- Acide Nalidixique 50 mg
- Colistine 5 mg
- Cristal Violet 0,5 mg
- 5-bromo-4-chloro-3-indoxyl-β-glucoside 50 mg

### Exemple 2

L'étalement de bactéries sur le milieu selon l'invention a donné les résultats suivants (24 heures d'incubation à 37°C).

| | Croissance | Couleur |
|---|---|---|
| Entérocoques | + | bleu-mauve |
| Staphylocoques | - | ---------- |

L'utilisation du milieu selon l'invention permet donc de détecter les entérocoques, et de les discriminer des staphylocoques.

### Exemple 3

Exemples de milieux pour entérocoques pouvant être utilisés dans le cadre de la présente invention, en y ajoutant du Cristal Violet et en supprimant éventuellement l'azide de sodium, ou en ajoutant de agents chromogènes, et éventuellement de la vancomycine, pour détecter les souches résistantes.

### Milieu bile-esculine (composition en grammes par litre) :

Extrait de viande : 3,0
Peptone de viande : 5,0
Bile de boeuf : 40,0
Esculine : 1,0
Citrate de fer : 0,5
Agar: 14,5
Ce milieu peut être enrichi de 5 p. cent de sérum de cheval.

### Milieu bile-esculine-azide (composition en grammes par litre) :

Tryptone : 17,0
Peptone : 3,0
Extraits de levure : 5,0
Esculine : 1,0
NaCl : 5,0
Citrate de fer ammoniacal : 0,5
Citrate de sodium : 1,0
Azide de sodium : 0,25
Bile de boeuf : 10,0
Agar : 13,5

### Milieu de Slanetz et Bartley ou m-Enterococcus agar (composition en grammes par litre) :

Tryptone : 15,0
Peptone : 5,0
Extraits de levure : 0,5
Glucose : 2,0 ou 5,0
K2HPO4 : 4,0
Azide de sodium : 0,4
Chlorure 2, 3, 5 triphényltétrazolium (solution à 1 p. cent) : 10,0
Agar : 10

### Milieu acide oxolinique - esculine - azide ou milieu OAA (composition en grammes par litre) :

Tryptone : 20,0
Extraits de levure : 5,0
Glucose : 1,0
NaCl : 5,0
Citrate de fer ammoniacal : 0,5
Citrate de sodium : 1,0
Azide de sodium : 0,4
Acide oxolinique : 0,005
Agar: 10,0

### Gélose esculine-azide-kanamycine (composition en grammes par litre) :

Tryptone : 20,0
Extraits de levure : 5,0
Glucose : 1,0
NaCl : 5,0
Citrate de fer ammoniacal : 0,5
Citrate de sodium : 1,0
Azide de sodium : 0,15
Sulfate de kanamycine : 0,02
Agar: 10,0

## Revendications

1. Milieu de culture pour la détection et/ou la discrimination des entérocoques, **caractérisé en ce qu'**il comporte, dans un milieu de culture pour entérocoques, du Cristal Violet à une concentration permettant la croissance des entérocoques et l'inhibition de croissance de la majorité des bactéries Gram positives, ladite concentration étant supérieure á 0,1 mg/l et inférieure à 1mg/l.

2. Milieu de culture selon la revendication 1, **caractérisé en ce qu'**il comprend en outre au moins un agent chromogène, substrat d'une enzyme de fermentation des sucres, choisi de telle sorte que le chromophore libéré possède une longueur d'onde différente de celle du Crystal Violet.

3. Milieu selon la revendication 2, **caractérisé en ce que** ladite enzyme est une glucosidase, notamment la β-glucosidase ou une galactosidase, notamment la β-galactosidase.

4. Milieu de culture selon la revendication 2 ou 3, **caractérisé en ce que** ledit agent chromogène libère, par hydrolyse, un chromophore précipitable choisi parmi les dérivés indoxyle, halogéno-indoxyle (bromo-indoxyle, chloro-indoxyle, fluoro-indoxyle, iodo-indoxyle, dichloro-indoxyle, chloro-bromo- indoxyle, tri-chloro-indoxyle), méthyl-indoxyle, ou hydroxy-quinoline, en particulier les dérivés suivants : 6-chloro-indoxyle, 5-bromo-indoxyle, 3-bromo-indoxyle, 6- fluoro-indoxyle, 5-iodo-indoxyle, 4,6-dichloro-indoxyle, 6,7-dichloro-indoxyle, 5-bromo-4-chloro-indoxyle, 5-bromo-6-chloro-indoxyle, 4,6,7-trichloro-indoxyle, N-méthyl-indoxyle ou 8-hydroxy-quinoline.

5. Milieu de culture selon la revendication 3 ou 4, **caractérisé en ce que** ledit substrat de la β-glucosidase est un indoxyl-glucoside, et/ou ledit substrat de la β-galactosidase est un indoxyl-galactoside.

6. Milieu de culture selon la revendication 5, **caractérisé en ce que** le substrat de la β-glucosidase est le 5-bromo-4-chloro-3-indoxyl-β-glucoside et/ou le substrat de la β-galactosidase est le 5-bromo-6-chloro-3-indoxyl-β-galactoside.

7. Milieu de culture selon l'une des revendications 1 à 6, **caractérisé en ce qu'**il contient également des inhibiteurs de croissance pour les bactéries Gram négatives.

8. Milieu de culture selon l'une des revendications 1 à 7, **caractérisé en ce qu'**il comprend (pour un litre) :
- Agar 15 g
- Extrait de levure et peptones 9 g
- NaCl 5 g
- Acide Nalidixique 50 mg
- Colistine 5 mg
- Cristal Violet 0,5 mg
- 5-bromo-4-chloro-3-indoxyl-β-glucoside 50 mg

9. Milieu de culture selon l'une des revendications 1 à 8, contenant en outre des antibiotiques.

10. Milieu de culture selon l'une des revendications 1 à 9, **caractérisé en ce qu'**il ne contient pas d'azide de sodium.

11. Utilisation d'un milieu de culture tel que défini dans l'une des revendications 1 à 10 pour la détection et/ou la discrimination des entérocoques.

12. Procédé de détection et/ou discrimination des entérocoques dans un échantillon, **caractérisé en ce qu'**il comprend les étapes consistant à :
a. inoculer un milieu de culture tel que défini dans l'une des revendications 1 à 10 avec ledit échantillon ou un inoculum dérivé de l'échantillon,
b. détecter la présence d'entérocoques sur ledit milieu de culture.

13. Milieu de culture pour la détection de bactéries Gram positives ou Gram négatives, comprenant, outre des facteurs de croissance pour lesdites bactéries Gram positives ou Gram négatives, un agent chromogène et du Cristal Violet, à une concentration permettant la croissance desdites bactéries que l'on cherche à détecter et l'inhibition différentielle de croissance des bactéries Gram positives, ladite concentration étant supérieure à 0.1mg/l et inférieure à 1mg/l ledit chromogène étant choisi de telle sorte que le chromophore libéré possède une longueur d'onde différente de celle du Crystal Violet.

## Patentansprüche

1. Kulturmedium für den Nachweis und/oder die Unterscheidung von Enterokokken, **dadurch gekennzeichnet, dass** es in einem Kulturmedium für Enterokokken Kristallviolett bei einer Konzentration umfasst, welche das Wachstum der Enterokokken und die Inhibierung des Wachstums des überwiegenden Teils von gram-positiven Bakterien ermöglicht, wobei die Konzentration über 0,1 mg/l und unter 1 mg/l liegt.

2. Kulturmedium nach Anspruch 1, **dadurch gekennzeichnet, dass** es darüber hinaus ein chromogenes Agens, Substrat eines Fermentationsenzyms von Zuckern, umfasst, das derart ausgewählt ist, dass der freigesetzte Chromophor eine andere Wellenlänge als jene des Kristallvioletts besitzt.

3. Medium nach Anspruch 2, **dadurch gekennzeichnet, dass** das Enzym eine Glucosidase, insbesondere β-Glucosidase, oder eine Galactosidase, insbesondere β-Galactosidase, ist.

4. Kulturmedium nach Anspruch 2 oder 3, **dadurch gekennzeichnet, dass** das chromogene Agens durch Hydrolyse einen fällbaren Chromophor freisetzt, der ausgewählt ist aus Indoxyl-, Halogenindoxyl- (Bromindoxyl-, Chlorindoxyl-, Fluorindoxyl-, Iodindoxyl, Dichlorindoxyl-, Chlorbromindoxyl-, Trichlorindoxyl-), Methylindoxyl- oder Hydroxychinolin-Derivaten, insbesondere den folgenden Derivaten: 6-Chlorindoxyl, 5-Bromindoxyl, 3-Bromindoxyl, 6-Fluorindoxyl, 5-Iodindoxyl, 4,6-Dichlorindoxyl, 6,7-Dichlorindoxyl, 5-Brom-4-chlorindoxyl, 5-Brom-6-chlorindoxyl, 4,6,7-Trichlorindoxyl, N-Methyl-indoxyl oder 8-Hydroxychinolin.

5. Kulturmedium nach Anspruch 3 oder 4, **dadurch gekennzeichnet, dass** das Substrat der β-Glucosidase ein Indoxylglucosid ist und/oder das Substrat der β-Galactosidase ein Indoxylgalactosid ist.

6. Kulturmedium nach Anspruch 5, **dadurch gekennzeichnet, dass** das Substrat der β-Glucosidase 5-Brom-4-chlor-3-indoxyl-β-glucosid ist und/oder das Substrat der β-Galactosidase 5-Brom-6-chlor-3-indoxyl-β-galactosid ist.

7. Kulturmedium nach einem der Ansprüche 1 bis 6, **dadurch gekennzeichnet, dass** es auch Wachstumsinhibitoren für gram-negative Bakterien enthält.

8. Kulturmedium nach einem der Ansprüche 1 bis 7, **dadurch gekennzeichnet, dass** es umfasst (pro Liter):
- Agar 15 g
- Hefeextrakt und Peptone 9 g
- NaCl 5 g
- Nalidixinsäure 50 mg
- Colistin 5 mg
- Kristallviolett 0,5 mg
- 5-Brom-4-chlor-3-indoxyl-β-glucosid 50 mg

9. Kulturmedium nach einem der Ansprüche 1 bis 8, das darüber hinaus Antibiotika enthält.

10. Kulturmedium nach einem der Ansprüche 1 bis 9, **dadurch gekennzeichnet, dass** es kein Natriumazid enthält.

11. Verwendung eines Kulturmediums, wie in einem der Ansprüche 1 bis 10 definiert, für den Nachweis und/oder die Unterscheidung von Enterokokken.

12. Verfahren zum Nachweis und/oder zur Unterscheidung von Enterokokken in einer Probe, **dadurch gekennzeichnet, dass** es die folgenden Schritte umfasst, welche bestehen aus:
a. Impfen eines Kulturmediums, wie in einem der Ansprüche 1 bis 10 definiert, mit der Probe oder einem Inokulum, das von der Probe abgeleitet ist,
b. Nachweisen der Anwesenheit von Enterokokken auf dem Kulturmedium.

13. Kulturmedium für den Nachweis von gram-positiven oder gram-negativen Bakterien, umfassend außer den Wachstumsfaktoren für die gram-positiven oder gram-negativen Bakterien ein chromogenes Agens und Kristallviolett bei einer Konzentration, welche das Wachstum der Bakterien, die man nachweisen will, und die differenzielle Inhibierung des Wachstums von gram-positiven Bakterien ermöglicht, wobei die Konzentration über 0,1 mg/l und unter 1 mg/l liegt, wobei das Chromogen derart ausgewählt ist, dass der freigesetzte Chromophor eine andere Wellenlänge als jene des Kristallvioletts besitzt.

## Claims

1. Culture medium for detecting and/or distinguishing enterococci, **characterized in that** it contains, in a culture medium for enterococci, Crystal Violet at a concentration allowing the growth of enterococci and the inhibition of the growth of most Gram-positive bacteria, said concentration being greater than 0.1 mg/l and less than 1 mg/l.

2. Culture medium according to Claim 1, **characterized in that** it additionally comprises at least one chromogenic agent, a substrate for an enzyme for sugar fermentation, chosen such that the chromophore released has a wavelength different from that of Crystal Violet.

3. Medium according to Claim 2, **characterized in that** said enzyme is a glucosidase, in particular β-glucosidase or a galactosidase, in particular β-galactosidase.

4. Culture medium according to Claim 2 or 3, **characterized in that** said chromogenic agent releases, by hydrolysis, a precipitable chromophore chosen from indoxyl, haloindoxyl (bromoindoxyl, chloroindoxyl, fluoroindoxyl, iodoindoxyl, dichloroindoxyl, chlorobromoindoxyl, trichloroindoxyl), methylindoxyl or hydroxyquinoline derivatives, in particular the following derivatives: 6-chloroindoxyl, 5-bromoindoxyl, 3-bromoindoxyl, 6-fluoroindoxyl, 5-iodoindoxyl, 4,6-dichloroindoxyl, 6,7-dichloroindoxyl, 5-bromo-4-chloroindoxyl, 5-bromo-6-chloroindoxyl, 4,6,7-trichloroindoxyl, N-methylindoxyl or 8-hydroxyquinoline.

5. Culture medium according to Claim 3 or 4, **characterized in that** said β-glucosidase substrate is an indoxylglucoside, and/or said β-galactosidase substrate is an indoxyl-galactoside.

6. Culture medium according to Claim 5, **characterized in that** the β-glucosidase substrate is 5-bromo-4-chloro-3-indoxyl-β-glucoside and/or the β-galactosidase substrate is 5-bromo-6-chloro-3-indoxyl-β-galactoside.

7. Culture medium according to one of Claims 1 to 6, **characterized in that** it also contains growth inhibitors for Gram-negative bacteria.

8. Culture medium according to one of Claims 1 to 7, **characterized in that** it comprises (for one litre) :
- Agar 15 g
- Yeast extract and peptones 9 g
- NaCl 5 g
- Nalidixic acid 50 mg
- Colistin 5 mg
- Crystal Violet 0.5 mg
- 5-bromo-4-chloro-3-indoxyl-β-glucoside 50 mg

9. Culture medium according to one of claims 1 to 8, additionally containing antibiotics.

10. Culture medium according to one of Claims 1 to 9, **characterized in that** it does not contain sodium azide.

11. Use of a culture medium as defined in one of Claims 1 to 10 for detecting and/or distinguishing enterococci.

12. Method for detecting and/or distinguishing enterococci in a sample, **characterized in that** it comprises the steps consisting in:
a. inoculating a culture medium as defined in one of Claims 1 to 10 with said sample or an inoculum derived from the sample,
b. detecting the presence of enterococci on said culture medium.

13. Culture medium for the detection of Gram-positive or Gram-negative bacteria comprising, in addition to growth factors for said Gram-positive or Gram-negative bacteria, a chromogenic agent and Crystal Violet, the Crystal Violet being present at a concentration allowing the growth of said bacteria which it is sought to detect and the differential inhibition of the growth of Gram-positive bacteria, said concentration being greater than 0.1 mg/l and less than 1 mg/l, said chromogenic agent being chosen such that the chromophore released has a wavelength different from that of Crystal Violet.
